Europäisches Patentamt

**European Patent Office**

Office européen des brevets

(19)

(11) Numéro de publication : **0 286 554 B1**

(12)

# FASCICULE DE BREVET EUROPEEN

(45) Date de publication du fascicule du brevet :
27.03.91 Bulletin 91/13

(51) Int. Cl.⁵ : **A61F 13/08, A61F 13/06**

(21) Numéro de dépôt : **88420107.0**

(22) Date de dépôt : **01.04.88**

(54) **Chevillère ligamentaire antivarus pour entorse en phase aigue.**

(30) Priorité : 03.04.87 FR 8704977

(43) Date de publication de la demande :
12.10.88 Bulletin 88/41

(45) Mention de la délivrance du brevet :
27.03.91 Bulletin 91/13

(84) Etats contractants désignés :
AT BE CH DE ES GB GR IT LI LU NL SE

(56) Documents cités :
EP-A- 0 114 560
US-A- 2 645 222
US-A- 3 777 751
US-A- 4 367 733

(73) Titulaire : ETABLISSEMENTS PICHON
FRERES, Société Anonyme
73, rue de la Tour
F-42000 Saint-Etienne (FR)

(72) Inventeur : Pichon, Jean Claude
8, allée Jean Gultton
F-42000 St Etienne (FR)

(74) Mandataire : Perrier, Jean-Pierre et al
Cabinet GERMAIN & MAUREAU 12 rue de la
République
F-42000 St-Etienne (FR)

## Description

L'entorse de la cheville est dans 95 % des cas une entorse externe et consiste en une lésion du ligament latéral externe qui comprend trois faisceaux :
 – un faisceau antérieur ou péronéo astragalien antérieur,
 – un faisceau moyen ou péronéo calcanéen,
 – un faisceau postérieur ou péronéo astragalien postérieur.

Le mécanisme est une inversion du pied soit pure (varus), soit avec une extension (varus équin), cas dans lequel il existe en plus une lésion capsulaire antérieure.

L'entorse externe est classée en bénigne, moyenne et grave, en fonction de la gravité de l'atteinte du ligament externe. La conduite à tenir est différente dans ces trois cas.

 – L'entorse bénigne correspond à un léger étirement ligamentaire au niveau du faisceau antérieur. Une contention élastique ou une chevillère est nécessaire pendant quelques jours. La reprise sportive s'effectue au bout de dix jours.

 – L'entorse de gravité moyenne comporte des signes locaux de gravité (oedème, hématome) sans laxité importante. Elle correspond à un étirement ou à la rupture de quelques fibres ligamentaires. La suppression de l'appui est nécessaire pendant quelques jours, une gouttière plâtrée peut être mise en place pendant une semaine si l'hématome et l'oedème sont importants ; puis une contention élastique ou une chevillère est mise en place, parfois une botte plâtrée de marche pendant quinze jours, s'il existe une lésion capsulaire antérieure. Une rééducation est prescrite.

 – L'entorse grave correspond à la rupture d'un ou plusieurs faisceaux du ligament latéral externe, le traitement est soit orthopédique, recours à un plâtre pendant quarante cinq jours, soit chirurgical. Il est suivi d'une rééducation avec port d'une chevillère.

L'instabilité chronique de la cheville est une pathologie de cause variée (séquelles d'entorse, hyperlaxité constitutionnelle). Elle entraine des épisodes d'instabilité et d'entorses à répétition. Le sujet n'est pas sûr sur sa cheville qui lâche plus ou moins fréquemment. Elle nécessite une rééducation adaptée, ainsi que la mise en place d'une chevillère de rééducation. La rééducation stimule les muscles péroniers latéraux pour que leur contraction soit plus adaptée et plus efficace, la chevillère supplée les ligaments distendus.

Il ressort de ce qui précède que la chevillère est un moyen important de contention et de suppléance ligamentaire et qu'elle doit être conçue pour assurer parfaitement cette fonction sans induire d'autres inconvénients tels qu'inconfort, pincement, irritations voire blessures cutanées par pression localisée consécutive au port d'une chaussure.

Il existe de nombreux types de chevillère. Les plus simples sont formées par une chaussette extensible assurant une contention généralisée de la partie lésée. En pratique de telles chevillères assurent un faible soutien des ligaments lésés et ont davantage un rôle préventif que curatif.

D'autres chevillères du type décrit dans le brevet américain 4 367 733 sont composées d'une chaussette extensible à talon ouvert associée à une bande élastique dont l'enroulement, de manière déterminée, autour du pied, forme plusieurs segments de bandes superposées et croisées qui, constituant élément de contention complémentaire, sont positionnés les uns par rapport aux autres par accrochage de fragments de velours à boucles et crochets. Lorsqu'une telle chevillère est en place sur le pied, elle assure une fonction de maintien proche de celle rigide procurée par un plâtre fermé que de celle souple procurée par une chevillère élastique. De plus, la superposition des fragments de bandes, entre eux et avec les moyens de positionnement, forme des surépaisseurs interdisant le port des chaussures et pouvant blesser l'utilisateur par pincement ou formation d'hématomes.

On connait également par le brevet européen 0 114 560 une chevillère de rééducation composée d'une chaussette extensible associée à une bande extensible dont la mise en place sur le pied forme des fragments de bandes qui, disposés en "Y" et parallèles aux faisceaux sous-jacents du ligament externe, compensent l'instabilité de ce ligament et permet le mouvement contrôlé de la cheville. Le positionnement des fragments de bandes fait aussi appel à des fragments de velours à boucles et crochets constituant des surépaisseurs locales pouvant entrainer une gène.

La présente invention décrite dans la revendication 1, délimitée par rapport à l'état de la technique la plus proches : US-A-4 367 733, a pour but de fournir une chevillère pour entorse en phase aigue du type précité, c'est à dire comprenant une chaussette extensible associée à une bande extensible qui, bloquant le pied en varus, permet la cicatrisation du ligament lésé et sans nécessiter de moyens d'accrochage complémentaires, et sans former de surépaisseurs gênantes.

Dans cette chevillère, du type comportant une bande extensible, la bande extensible est fixée sur la partie médiane de la chaussette extensible, en partie sur l'élément tubulaire supérieur et en partie sur l'élément tubulaire inférieur de la chaussette, et comporte une extrémité libre en forme de pointe sur les bords de laquelle sont fixées les extrémités d'une bande élastique formant une boucle apte à s'accrocher sans plissage sur le talon, après passage de la bande à l'intérieur du pied, sous le coup de pied, sur le coup de pied en chevauchant le faisceau antérieur du ligament latéral externe, puis contre la malléole interne.

Lorsque cette chevillère est mise en place, la boucle de sa bande maintient parfaitement cette dernière sans blesser le porteur et assure ainsi le bon positionnement du fragment de bande qui, chevauchant le faisceau antérieur du ligament latéral externe, renforce l'action de ce faisceau antérieur, soulage celui du faisceau moyen du même ligament externe, lutte contre le varus du pied et contre tout étirement ligamentaire, sans s'opposer à l'extension du pied.

Cette chevillère extensible procure les mêmes effets qu'une contention élastique collée, maintenant le pied à angle droit et en léger valgus, tout en apportant un plus grand confort d'utilisation et une mobilité en extension.

D'autres caractéristiques et avantages ressortiront de la description qui suit en référence au dessin schématique annexé représentant à titre d'exemples non limitatifs une forme d'exécution de cette chevillère.

Figure 1 est une vue de côté de la chevillère seule,

Figures 2, 3 et 4 sont des vues en perspective montrant la chevillère lorsqu'elle est placée sur une cheville droite et dans différentes phases d'enroulement de sa bande extensible,

Figure 5 est une vue en perspective montrant, de l'intérieur du pied, la chevillère posée.

De façon connue, cette chevillère est composée d'une chaussette à talon ouvert, désignée de façon générale par 2, et d'une bande extensible 3. La chaussette, réalisée en matériau extensible, est composée d'un élément tubulaire supérieur 2a et d'un élément tubulaire inférieur 2b. Ces deux éléments sont reliés sur la face antérieure de la cheville pour former une chevillère à talon ouvert. La bande extensible 3 est fixée par son extrémité 3a sur la partie médiane de la chevillère, c'est à dire en partie sur l'élément tubulaire 2a et en partie sur l'élément tubulaire 2b. Dans la forme d'exécution représentée, cette répartition est égale.

L'extrémité libre 3b de la bande extensible 3 est en forme de pointe. Ses bords 4 délimitent un angle au sommet a compris entre 110 et 120°. Sur chacun de ses bords 4, sont fixées les extrémités d'une bande élastique de plus petite largeur que celle 3 et formant une boucle 5.

Il est évident que les dimensions de la chevillère, la largeur et la longueur de la bande 3, de même que la largeur et la longueur de la boucle 5, sont adaptées à la morphologie de l'utilisateur en donnant diverses tailles à la chevillère.

Lorsque la chevillère est enfilée sur la cheville, l'élément supérieur 2a de la chaussette extensible assure un maintien ferme de la mortaise tibio-péronière, tandis que son élément inférieur 2b assure un maintien ferme du tarse postérieur et ceci grâce à une forte contention du tissu extensible constituant la chaussette. La solidarité des deux éléments au niveau antérieur de l'articulation assure une stabilisation articulaire.

En raison de la fixation de la bande 3 sur la partie médiane de la chevillère, la même chevillère peut être utilisée indifféremment pour une entorse de pied droit ou pour une entorse de pied gauche.

Cette fixation est effectuée par couture et de façon à former une très faible surépaisseur, sans création de bourrelet saillant susceptible de blesser le coup de pied.

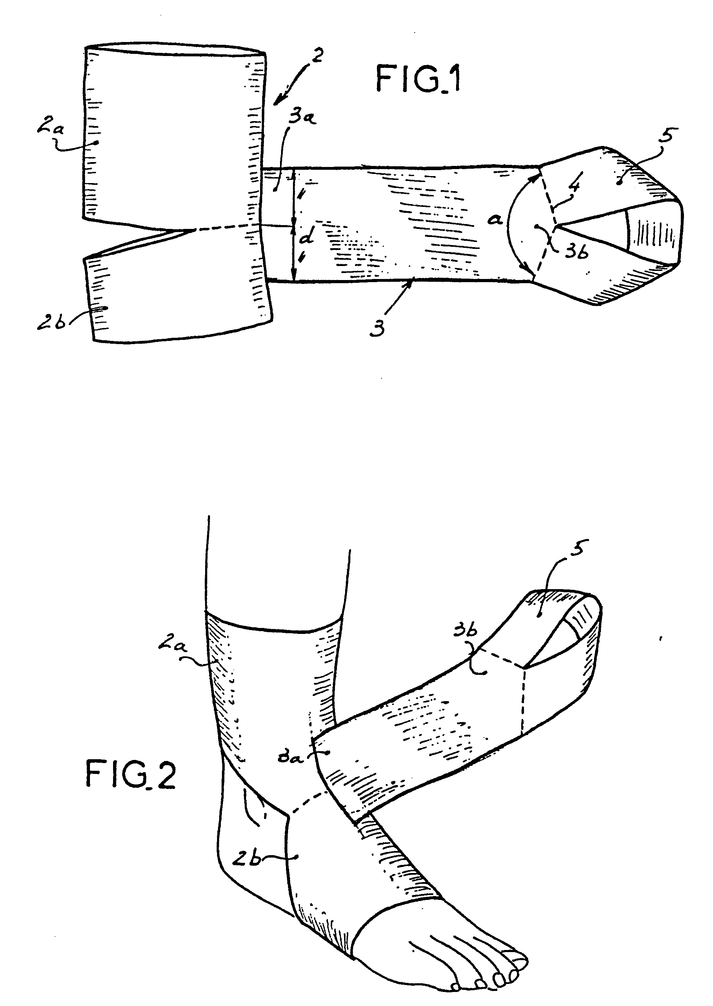

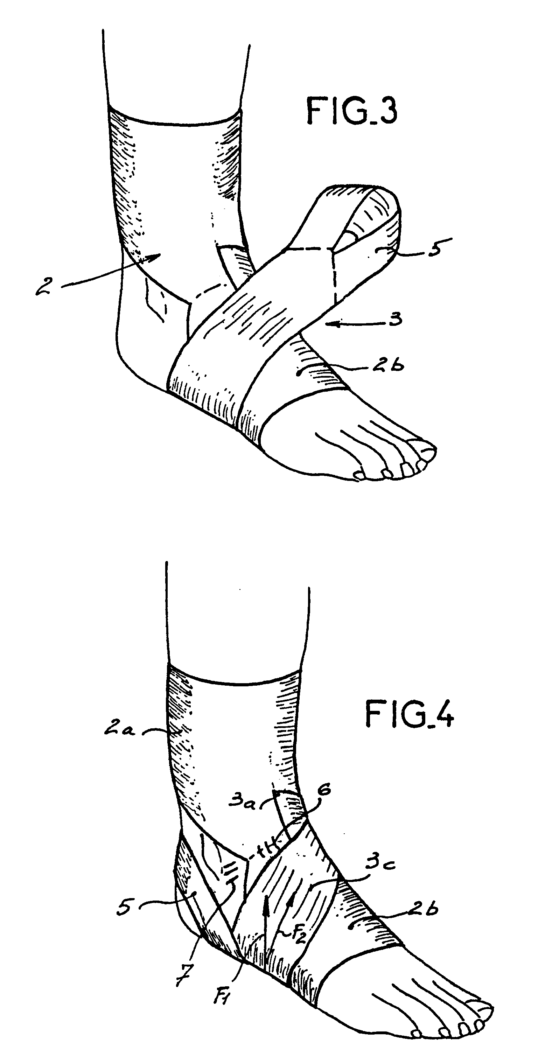

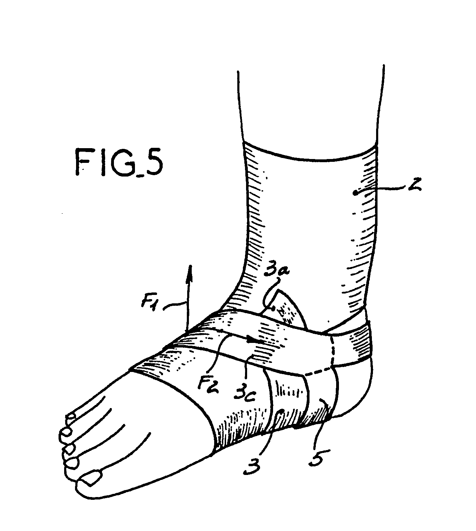

La mise en place de la chevillère est terminée en faisant passer la bande à l'intérieur du pied, comme représenté à la figure 2, en l'amenant vers l'extérieur sous la voûte plantaire, en la ramenant sur le coup de pied, comme montré à la figure 3, et en l'amenant vers la malléole interne (figure 5), de manière à permettre l'accrochage de la boucle 5 sur le talon, comme montré à la figure 4.

La figure 5 montre que la bande enveloppe le coup de pied et vient contre la malléole interne, où elle est prolongée par la boucle qui s'accroche derrière et sous le talon.

Il est à noter que grâce à la forme de la boucle, celle-ci ne plisse pas lorsqu'elle est positionnée sur le talon. Elle assure un excellent maintien de l'ensemble de la bande, sans pour autant engendrer une surépaisseur gênante pour l'utilisateur puisqu'elle se positionne dans l'ouverture du talon de la chaussette.

Le fragment de bande 3c visible à la figure 4 chevauche le faisceau antérieur 6 et est sensiblement parallèle au faisceau moyen 7 du ligament latéral externe. Il exerce sur le pied des forces, respectivement verticales F1 et transversales F2. La force F1 soulève le bord externe du pied en le tirant vers le haut tandis que celle F2 le tire en valgus, c'est à dire s'oppose au varus. Ces deux forces soulagent les faisceaux antérieur 6 et latéral 7 du ligament externe et permettent leur cicatrisation. Dans ces conditions, la bande s'oppose au varus du pied et à tout étirement ligamentaire. Elle reproduit ainsi l'effet que l'on recherche lorsque l'on met en place une contention élastique collée, contention maintenant le pied en angle droit et en léger valgus.

Cette bande supplée le ligament défaillant en cas de laxité chronique et coapte l'articulation en lui laissant une faible liberté de mouvement, tout en assurant un rappel dynamique lors de tout baillement excessif.

Les indications de cette chevillère découlent de son effet anti varus.

Dans l'entorse bénigne, elle est mise en place pendant les dix premiers jours pour permettre la cicatrisation du ligament, puis lors de la reprise sportive pendant quelques jours.

Dans l'entorse de gravité moyenne, elle est indiquée immédiatement si les signes locaux (oedème, hématome) ne sont pas trop importants, sinon huit jours après la mise en place d'une gouttière plâtrée et du sevrage d'appui. Elle est laissée pendant toute la phase de rééducation et de réadaptation sportive.

Dans l'entorse grave, elle est présente après chirurgie, ou plâtre de quarante cinq jours, c'est à dire pendant la phase de rééducation et de réadaptation sportive.

## Revendications

1. Chevillère ligamentaire anti varus du type composé d'une chaussette extensible (2), à talon ouvert, solidaire de l'extrémité d'une bande extensible (3) munie à son autre extrémité de moyens d'accrochage, caractérisée en ce que la bande extensible (3) est fixée sur la partie médiane de la chaussette extensible (2), en partie sur l'élément tubulaire supérieur (2a) et en partie sur l'élément tubulaire inférieur (2b) de cette chaussette, et comporte une extrémité libre (3b), en forme de pointe, sur les bords (4) de laquelle sont fixées les extrémités d'une bande élastique (5) formant une boucle apte à s'accrocher sans plissage sur le talon, après passage de la bande à l'intérieur du pied, sous le coup de pied, sur le coup de pied, en chevauchant le faisceau antérieur du ligament latéral externe, puis contre la malléole interne.

2. Chevillère selon la revendication 1 caractérisée en ce que l'angle au sommet (a) de l'extrémité en pointe (3a) de la bande extensible (3) est comprise entre 110 et 120°.

## Ansprüche

1. Anti-Varus-Knöchelbandage des zusammengesetzten Typs aus einem dehnbaren Strumpf (2), der an der Ferse offen und mit dem Ende eines dehnbaren Bandes (3) verbunden ist, das an seinem anderen Ende mit Anhängemitteln versehen ist, **dadurch gekennzeichnet,** daß das dehnbare Band (3) auf dem mittleren Abschnitt des dehnbaren Strumpfes (2) teilweise auf dem rohrförmigen oberen Element (2a) und teilweise auf dem rohrförmigen unteren Element (2b) dieses Strumpfes befestigt ist und ein freies Ende (3b) in Form einer Spitze aufweist, auf deren Rändern (4) die Enden eines elastischen Bandes (5) befestigt sind, das nach dem Verlauf des Bandes an der Innenseite des Fußes, unter dem Mittelfuß, auf dem Mittel.fuß, das vordere Bündel des äußeren Seitenbandes übergreifend und dann gegen den inneren Fußknöchel eine zum faltenlosen Anhängen auf die Ferse geeignete Schlinge bildet.

2. Bandage nach Anspruch 1, **dadurch gekennzeichnet,** daß der Winkel am Scheitelpunkt (a) des Endes an der Spitze (3b) des dehnbaren Bandes (3) zwischen 110° und 120° beträgt.

## Claims

1. An anti-varus ligamentary ankle band comprising a stretch sock (2), having an open heel, connected to the end of a stretch band (3) provided at its other end with fixing means, characterised in that the stretch band (3) is fixed to the median part of the stretch sock (2), partly on the upper tubular element (2a) and partly on the lower tubular element (2b) of this sock, and has a free end (3b) in the form of a point, on the edges (4) of which are fixed the ends of an elastic band (5) forming a loop adapted to be fixed without folding on the heel, after passing the band around the inside of the foot, under the instep, over the instep, straddling the front bundle of fibres of the lateral external ligament, then against the inside of the malleolus.

2. An ankle band according to Claim 1, characterised in that the angle at the apex (a) of the pointed end (3a) of the stretch band (3) is between 110° and 120°.

FIG. 1

2

2a

3a

5

4

d

a

3b

3

2b

FIG. 2

2a

5

3b

3a

2b

FIG.3

2

5

3

2b

FIG.4

2a

3a    6

th

3c

5

2b

7

F₂

F₁

FIG_5